# EUROPEAN PATENT SPECIFICATION

(11) **EP 3 548 103 B1**
(45) Date of publication and mention of the grant of the patent: **14.02.2024**
(21) Application number: 17786885.8
(22) Date of filing: 11.10.2017
(51) Int. Cl.: A61L 15/24, G01N 33/52

(54) **ANTIPERSPIRANT DEVICE AND METHOD**
SCHWEISSHEMMENDE VORRICHTUNG UND -VERFAHREN
DISPOSITIF ET PROCÉDÉ ANTIPERSPIRANTS

(30) Priority: 02.11.2016 EP 16196859; 02.11.2016 EP 16196872
(43) Date of publication of application: 09.10.2019
(73) Proprietor: Unilever IP Holdings B.V., 3013 AL Rotterdam (NL); Unilever Global IP Limited, Wirral, Merseyside CH62 4ZD (GB)
(72) Inventor: BATES, Susan, Bebington Wirral Merseyside CH63 3JW (GB); HADDLETON, David, Mark, Coventry Warwickshire CV4 7AL (GB); HAND, Rachel, Alice, Coventry Warwickshire CV4 7AL (GB); KHOSHDEL, Ezat, Bebington Wirral Merseyside CH63 3JW (GB)
(74) Representative: Whaley, Christopher
(86) International application number: PCT/EP2017/075993
(87) International publication number: WO 2018/082881

(56) References cited:
- WO-A1-00/69405
- WO-A1-02/087645
- WO-A1-2006/111748
- WO-A2-2008/154546
- WO-A2-2009/019485
- US-A- 5 441 048

## Description

### Field of Invention

The present invention is in the field of cosmetic products, in particular devices for adsorbing sweat and malodour.

### Background

The surface of the human body, i.e. the skin, produces sweat as part of the body's thermoregulatory function and in response to emotional and/or physical stress. Unfortunately, these natural secretions can result in problems for the individual affected; for example, the sweat may result in wet patches on clothing and often leads to the production of body odour.

For many years, sweating or perspiration has been treated with antiperspirant compositions that reduce sweat production by partial blockage of the sweat glands. Whilst these compositions can be highly effective, they are rarely able to prevent egress of perspiration completely. In addition, some individuals do not like the thought of having their sweat glands obstructed.

A few inventors have put forward an alternative approach to antiperspirancy involving the absorption of perspiration, some examples of which are detailed below.

WO 2009/063016 (Unilever, 2009) discloses the use of emulsions that form a metastable amphiphile phase during application to the skin and which is able to adsorb perspiration.

EP 550,960 A1 (Unilever, 1992) discloses antiperspirant actives which form, upon contact with perspiration, a water-insoluble phase of greater than one-dimensional periodicity, this phase being capable of holding absorbed perspiration.

JP 5072362 B4 (Shiseido, 1993) discloses cosmetic compositions comprising a polymer having high water absorption properties and its use to absorb sweat.

WO 2003/026608 (P&G, 2003) discloses topical compositions comprising fluid absorbent solids for use in absorbing sweat and sebum.

WO 2005/102255 A2 (Coty, 2005) discloses deodorant compositions comprising a sweat-absorbing complex.

WO2008/154546 A2 (Avery Dennison Corp., 2008) discloses perspiration/odour absorbing patch with a general statement concerning hydrogels; however, it does not disclose the hydrogels used in the present invention.

WO2006/11748 A1 (Univ. Newcastle, 2006) discloses the sampling of volatiles found in sweat; however, hydrogels are not discussed.

US 5,441,048 A1 (Schoendorfer, 1995) discloses a system for determining an analyte in perspiration; however, there is no disclosure of the use of hydrogels.

The use of patches comprising a hydrogel layer for controlling perspiration has not been previously disclosed, to the best of our knowledge. However, such patches have been applied to the human skin for a variety of other reasons.

WO2000/69405 A1 (Lectec Corp., 2000) discloses a patch for treating acne, but pendant hydrophilic groups are not mentioned.

WO2002/087635 A1 (Av Topchiev Inst. Petrochemical, 2002) discloses wound dressings comprising hydrogels; however, hydrogels with pendant hydrophilic groups are not disclosed.

WO2009/019485 A2 (First Water Ltd., 2009) discloses a wound dressing using a hydrogel with pendant sulphonyl groups.

US 2012/0114591 A1 (First Water Ltd., 2012) discloses the use topical hydrogel treatment of wounds, the hydrogel comprising a hydrophilic polymer carrying multiple pendant sulphonyl groups.

US 2012/0116279 A1 (First Water Ltd., 2012) discloses the use of a multilayer dressing for wounds, the dressing including a gel layer in contact with the wound.

### Summary of the Invention

In a first aspect of the invention, there is provided a method of capturing perspiration from the human skin, said method comprising the topical application of a patch comprising an absorbent hydrogel layer and a backing sheet adhering to it, the perspiration being absorbed into the hydrogel layer and thereby trapped, wherein the hydrogel layer comprises a hydrophilic polymer which is an addition polymer having pendant hydrophilic groups.

In a second aspect of the invention, there is provided a method of capturing perspiration from the human skin and for releasing fragrance, said method comprising the topical application of a patch comprising an absorbent hydrogel layer having a benefit agent embedded therein and a backing sheet adhering to the hydrogel layer, the perspiration being absorbed into the hydrogel layer and the benfit agent being released thereby, wherein the hydrogel layer comprises a hydrophilic polymer which is an addition polymer having pendant hydrophilic groups.

It is an object of the present invention to provide an improved method for entrapping and removing perspiration from the surface of the human body, i.e. the skin, in particular the underarm regions thereof.

It is a further object of the present invention to deliver a deodorancy benefit to the surface of the human body. The deodorancy benefit may result from the entrapment of volatile malodourous materials together with the perspiration and/or the delivery of fragrance (*vide infra*)*.* This object of the invention may also be delivered together with the entrapment and removal of perspiration, as referred to above.

In certain embodiments, a further objection of the present invention is the delivery of a benefit agent, in particular a fragrance, to the surface of the human body.

Other benefits of the present invention include the ability of the patch to absorb sweat directly from the skin over short/long periods of time (minutes to hours) without occluding the skin surface, and with no transfer of adhesive to the skin.

### Detailed Description of the Invention

Herein, all percentages, parts and ratios are to be understood to be by weight, unless otherwise stated.

Herein, all amounts, percentages and ratios are to be understood as prefixed by the word "about".

Herein, any component, ratio or weight indicated as "preferred" is to be understood as preferably used in combination within any other component, ratio or weight indicated as "preferred".

Herein, the terms "comprising" and "comprised of", etc., are to be understood as meaning that other components/features could also be present; i.e. the listed steps or options need not be exhaustive.

Herein, the "inner" part/layer/section of the patch is that part/layer/section closer or closest to the skin when the patch is in use and the "outer" part/layer/section of the patch is that part/layer/section farther or farthest from the skin when the patch is in use.

Herein, "breathable" means that air is able to pass completely through the material at ambient conditions.

Herein, "permeable" means that fluids, in particular water, is able to pass through the material, at least in part, by wicking or by any other means at ambient conditions.

Herein, "ambient conditions" mean 25°C and one (1) atmosphere pressure and ambient conditions are to be understood to be prevalent unless otherwise stated.

Herein, "volatile" means having a significant vapour pressure (e.g. greater than 5 Pa or 10 Pa) at 25°C.

Herein, all the methods according to the invention are to be understood to be cosmetic, i.e. non-therapeutic, methods of treatment. Similarly, all products according to the invention are to be understood as intended for use in cosmetic, non-therapeutic, methods of treatment.

### Hydrogel layer

An absorbent hydrogel layer is an essential component of the present invention. The hydrogel layer needs to be in direct or indirect contact with the skin during the use of the patch in order to allow the volatile malodourous materials present of the skin to be absorbed into the hydrogel layer.

Herein, the term "hydrogel" is not to be considered as limited to gels that contain water, but to extend generally to all hydrophilic gels, including those containing organic non-polymeric components, with the requirement that the hydrogel layer comprises a hydrophilic polymer which is an addition polymer having pendant hydrophilic groups.

The hydrogel may be selected from non-ionic, anionic, cationic and zwitterionic natural hydrophilic biopolymers, synthetic hydrophilic polymers, hydrocolloids, gelling hydrophilic biopolymers and all combinations thereof, with the requirement that the hydrogel layer comprises a hydrophilic polymer which is an addition polymer having pendant hydrophilic groups.

Hydrogels are, generally speaking, hydrophilic polymers characterised by their ability to absorb large amounts of aqueous fluid without dissolving in said fluid.

The hydrogel hydrophilicity is generally due to groups such as hydroxyl, carboxyl, carboxamido, and esters. On contact with water, the hydrogel assumes a swollen hydrated state that results from a balance between the dispersing forces acting on hydrated chains and cohesive forces that hold the hydrogel together, but do not prevent the penetration of water into the polymer network. The cohesive forces are most often the result of crosslinking via covalent bonds, but may result from weak interactions such as electrostatic, hydrophobic or dipole-dipole interactions.

The hydrogel layer is a coherent, three-dimensional polymer capable of absorbing water without liquefying, i.e. it is insoluble in water. Usually, the insolubility in water is provided by crosslinking of a "base" polymer, although certain hydrogel are by their nature "crosslinked", for example, when the crosslinking occurs during the biosynthesis of the hydrogel.

Herein, the "base" polymer for the hydrogel layer is to be considered the total polymer in the theoretical absence of any crosslinking agent. In practice, the "base polymer" may never be formed; however, it may still be considered present in the hydrogel as the sum of the non-crosslinking monomers used in the synthesis.

The absorption of the volatile malodourous materials into the hydrogel layer is as part of the perspiration of which they are components and typically occurs by wicking of the perspiration into the hydrogel. This wicking is aided by the hydrogel comprising an hydrophilic polymer, in particular a hydrophilic polymer that is an addition polymer having pendant hydrophilic groups.

Suitable hydrophilic polymers for use as the hydrogel layer are gelatin, polysaccharides, crosslinked acrylamide polymers, thermoplastic polyurethanes (TPUs), crosslinked acrylates or methacrylates, crosslinked polymers and copolymers of N-vinylpyrrolidinone and crosslinked polymers and copolymers of acrylic acid, with the requirement that the hydrophilic polymer is an addition polymer having pendant hydrophilic groups.

Particularly suitable crosslinked acrylate and methacrylate hydrogels are ones comprising monomers selected from hydroxyethylacrylate, hydroxyethylmethacrylate, 2-(N,N-dimethylamino)ethyl methacylate and methacryloyloxyalkyl sulfonates (generally crosslinked with di-acrylate or di-vinylbenzene).

Particularly suitable crosslinked acrylamide hydrogels are ones comprising monomers selected from poly(2-acrylamido-2-methylpropane sulfonic acid) and salts thereof.

Hydrogels are described in greater detail in Hydrogels, Kirk-Othmer Encyclopedia of Chemical Technology, 4th Edition, vol. 7, pages 783-807, John Wiley and Sons, New York.

Preferred hydrophilic polymers for use in the present invention are metal salts of crosslinked of poly(2-acrylo-2-methyl-1-propanesulfonic acid) (PolyAMPS) and poly(2-hydroxyethylmethacrylate). A particularly preferred hydrophilic polymer is crosslinked sodium poly(2-acrylo-2-methyl-1-propanesulfonate). The non-neutralised base monomer and polymer [poly(2-acrylo-2-methyl-1-propanesulfonic acid^{®}] are available from Lubrizol Corp.

An especially preferred hydrogel for use in accordance with the present invention is sodium poly(2-acrylo-2-methyl-1-propanesulfonate) crosslinked with poly(ethylene glycol) diacrylate.

Differing techniques may be used to cause cross-linking of the hydrogel layer. Cross-linking may occur through condensation polymerization of monomers having multiple functionality or by covalent bonding between polymeric chain by techniques such irradiation, sulphur vulcanization, or other chemical.

The synthesis of the hydrogel is preferably a free radical polymerisation with cross-linking, which may, for example, be induced by light, heat, radiation (e.g. ionising radiation), or redox catalysis. The precursor solution may include appropriate initiators, as generally known in the art.

Crosslinking also can be carried out by reacting biopolymers or synthetic polymers with di- or multi-functional reactive molecules. For example, taking a polysaccharide or polyvinyl alcohol and reacting it with a reactive molecule such as epichlorohydrin, a polyacid, polyanhydride, or di-isocynates, can produce a cross-lined hydrogel.

Another example of cross-linking is to take a poly(acrylic acid) or carrageenan and crosslink it with polyethylene glycol or a Jeffamine.

Multi-functional monomers, in particular di-functional monomers, are suitable cross-linking agents used in the preparation of the hydrogel layer. Such monomers can impart crosslinking or branching sites to give the hydrogel its 3-dimensional "architecture".

Suitable di-functional cross-linking monomers are 2,2-bis[4-(2-acryloxyethoxy) phenyl] propane, *bis*(2-methacryloxyethyl)-N,N'-1,9-nonylene biscarbamate, 2,2-*bis*(4-methacryloxyphenyl) propane, 2,2-*bis*[4-(2-hydroxy-3-methacryloxy- propoxy)phenyl] propane, 1,4-butanediol diacrylate, 1,3-butanediol dimethacrylate, 1,4-butanediol dimethacrylate, trans-1,4-cyclohexanediol dimethacrylate, N,N'-cystaminebisacrylamide, 1,10-decanediol dimethacrylate, 1,4-diacryloylpiperazine, N,N'-diallylacrylamide, diethylene glycol diacrylate, 2,2-dimethylpropanediol dimethacrylate, dipropylene glycol dimethacrylate, N,N'-ethylene bisacrylamide, ethylene glycol diacrylate, ethylene glycol dimethacrylate, N,N'-hexamethylenebisacrylamide, 1,6-hexanediol diacrylate, N,N'-methylenebisacrylamide, nonanediol dimethacrylate, 1,5-pentanediol dimethacrylate, 1,4-phenylene diacrylate, tetraethylene glycol dimethacrylate, triethylene glycol diacrylate, triethylene glycol dimethacrylate and zinc dimethacrylate.

Suitable tri-and tetra-functional and cross-linking monomers are 1,1,1-trimethylolpropane triacrylate, 1,1,1-trimethylolpropane trimethacrylate, dipentaerythritol penta-acrylate (mixture of tetra-, penta- and hexa-acrylates) and pentaerythritol tetra-acrylate (mixture of tri- and tetra esters).

A particularly suitable crosslinking agent for the hydrogel layer is methylene-*bis*acrylamide, when the base polymer of an acrylamide derivative such as 2-acrylamido-2-methylpropanesulfonic acid or one of its salts.

Other particularly suitable crosslinking agent for the hydrogel layer are -*bis*-acrylates and *bis*-methacrylates, particularly when the base polymer is an acrylate or methacrylate derivative, for example, poly(2-hydroxyethylmethacrylate).

Generally, the level of crosslinking agent relative to the base polymer is quite low, usually equating to a weight ratio of less than 1:50. Preferably, this ratio is from 1:500 to less than 1:50 and more preferably, it is from 1:400 to 1:100. For the avoidance of doubt, the ratio of crosslinking agent to base polymer should be understood to equate to the ratio of crosslinking monomers to non-crosslinking monomers used in preparation of any such hydrogel.

The hydrophilic polymer part of the hydrogel is general its major component and preferably comprises greater than 80% of said layer. In some preferred embodiments, the hydrophilic polymer is the sole component of the hydrogel layer, other than the crosslinking agent that may be present therein.

In preferred embodiments, the hydrogel layer comprises one or more organic plasticisers that can serve to enhance the flexibility of the hydrogel. Suitable plasticisers may comprise any of the following either alone or in combination: at least one polyhydric alcohol (such as glycerol, polyethylene glycol, or sorbitol), at least one ester derived therefrom, at least one polymeric alcohol (such as polyethylene oxide) and/or at least one mono- or poly-alkylated derivative of a polyhydric or polymeric alcohol (such as alkylated polyethylene glycol). Glycerol is a preferred plasticiser. An alternative preferred plasticiser is the ester derived from boric acid and glycerol. When present, the organic plasticiser may comprise up to about 45% of the hydrogel layer, but is preferably from 1 to 30% and more preferably 1 to 10% thereof.

Any compatible surfactant or combination thereof may advantageously be used as an additional ingredient of the hydrogel layer. Such surfactants can lower the surface tension of the mixture before polymerisation and thus aid processing. The surfactant may be non-ionic, anionic, zwitterionic or cationic. The surfactant may itself be reactive, i.e. capable of participating in the hydrogel-forming reaction. The total amount of surfactant, if present, is suitably up to about 10% by weight of the hydrogel, but is preferably from 0.05% to 4% thereof.

In a preferred embodiment of the invention, the surfactant is at least one propylene oxide/ethylene oxide block copolymer, for example that supplied by BASF Plc under the trade name Pluronic^{®} P65 or L64.

The hydrogel layer typical has a sticky nature/feel. This serves to enhance the adhesion of the hydrogel layer to the skin and is therefore a preferred feature.

The sticky feel can, however, lead to sensory negatives with regard to its contact with the skin, a problem that can be alleviated by the presence of a permeable inner membrane, which is held between the hydrogel layer and the skin when the patch is in use (*vide infra*)*.*

The sticky nature typical of the hydrogel layer can also give problems with regard to any clothing worn by the wearer of the patch, in that the clothing may stick to the hydrogel layer and cause discomfort. This problem is addressed by the backing layer for the patch (*vide infra*)*.*

The absorbent nature of the hydrogel layer means that is also permeable, as defined herein.

It is preferred that the hydrogel layer is breathable, particularly in conjunction with the backing layer being breathable and especially in conjunction with any further components also being breathable. Having breathable components leads to comfort in use advantages, enabling the skin the keep close to the temperature and humidity levels of its surroundings. In addition, the breathability can help maintain the natural microflora present on the skin and thereby avoid any changes in odour that could otherwise result from their perturbation.

It is preferred that the hydrogel layer is flexible, for comfort in use benefits. Indeed, it is preferred that all components of the patch are sufficient flexible to allow it to flex with the movement of the skin of the body to which it is attached.

The surface area of the hydrogel layer can be tailored to meet the needs of the specific skin site to which it is to be applied. In addition, it must be borne in mind that multiple smaller areas of hydrogel layer may sometimes be used as an alternative to a single larger area. Thus, the surface area of hydrogel layer can be from 1 to 100 cm², although it is preferably from 8 to 80 cm² and more preferably 40 to 70 cm², especially when it is intended for a single patch to be applied to each single axilla of an individual requiring treatment.

In the aspects of the present invention relating to a "method", the method preferably involves the application of a single patch to each axilla of the individual requiring treatment.

In the aspects of the present invention relating to a "patch", it is preferably intended that each patch is suitable for and intended for application to each single axilla of the individual requiring treatment.

The thickness of the hydrogel layer may also be tailored to fit needs and may be from 1 micron to 1 cm; however, it is preferably from 0.5 to 6 mm and more preferably from 1 to 3 mm.

The water content of the hydrogel layer (immediately following manufacture of the patch) is preferably from 0 to 95%, more preferably from 0 to 60% and most preferably from 0 to 40% by weight, relative to the total weight of the hydrogel (including any water present).

The absorptive capacity of the hydrogel layer, as defined by its ability to absorb water at ambient temperature, is preferably from 100 or 200% by volume up to 400 or 500% by volume, based on the dry (i.e. zero water) volume of the hydrogel.

### Backing Layer

A backing layer for the hydrogel layer, present on its outer surface, is another essential component of the present invention. It main benefit, as mentioned above, is the avoidance of comfort-in-use issues that could otherwise result by the hydrogel layer sticking to any clothing worn by the wearer of the patch. Another benefit is that the backing layer may strengthen the associated hydrogel layer and thereby reduce damage to it, a particular problem in its most common location of use in the axillae.

It is preferred that the backing layer is flexible, for comfort in use reasons (*vide infra*)*.*

It is preferred that the backing layer is breathable, for the same reasons as referred to with regard to the hydrogel reason.

It is preferred that the backing layer covers the majority, i.e. greater than 50% by area of the outer surface of the hydrogel layer. It is particularly preferred that the backing layer covers greater than 90% by area of the outer surface of the hydrogel layer.

In certain preferred embodiments, it is preferred that the backing layer overlaps the outer surface of the hydrogel layer and is sufficiently flexibility and overlapping to itself contact the skin when the patch is in operation. This feature is particularly advantageous when the backing layer has adhesion to the skin that is greater than that of the hydrogel layer.

The backing layer may comprise poly(alkoxyalkyl)acrylate (e.g. poly(2-hydroxyethyl)acrylate) or poly(alkoxyalkyl)methacrylate (e.g. poly(2-hydroxyethyl)methacrylate) or polyurethane. It is preferred that the backing material comprises a polyurethane elastomer and it is particularly preferred that this is the predominate material in the backing material. The polyurethane may be of an ester or ether based grade.

Suitable backing materials include the polyurethane elastomers available under the registered trademark ESTANE^{®} or Pellethane^{®}, both available from Lubrizol Corp.

An example of a suitable Estane^{®} is Estane^{®} 58315, an 85A aromatic polyether-based thermoplastic polyurethane (TPU) elastomer.

An example of a suitable Pellethane^{®} is Pellethane^{®} 5863-80A TPU, an aromatic polyether-based TPU elastomer.

### Pressure Sensitive Adhesive

The backing layer may additionally comprise a pressure sensitive adhesive (PSA), particularly on its inner surface. This is particularly advantageous when the hydrogel layer has a permeable membrane on its inner surface. When the hydrogel layer has a permeable membrane on its inner surface, this serves to separate it from the skin in use and may reduce its adhesion thereto. In such circumstances, giving the backing layer a PSA on its inner surface and giving it sufficiently flexibility and overlap to contact the skin when the patch is in operation can overcome this problem. Hence, the presence of a PSA on the inner surface of the backing layer, together with the presence of a permeable membrane on its inner surface and the backing layer having sufficiently flexibility and overlap to contact the skin when the patch is in operation is a desired combination of features.

Herein, a PSA is an adhesive that forms a bond between the substrates with which it is in contact when pressure is applied. Further, it can be detached without leaving traces.

When employed, the PSA is preferably an acrylic polymer, a polyvinyl ethyl ether or a triblock rubber copolymer of styrene with butadiene or isoprene, the triblock copolymer being formulated with oil. The acrylic polymers are preferably solvent-based, in particular water-based emulsions. Preferred acrylics are acrylic esters, such as 2-ethylhexyl acrylate and ethyl acrylate. The triblock rubber copolymers are preferably formulated with a tackifier as well as an oil.

Particularly suitable PSAs are DURO-TAK^{™} PSAs available from Henkel GmbH and LEVAGEL^{®} (a polyurethane PSA) available from Dow Corning.

### Tackifier

Herein, a tackifier is a chemical compound used to increase the "tack", i.e. stickiness of the surface of an adhesive. Preferred tackifiers are resins, in particular rosins and their derivatives; terpenes and modified terpenes; aliphatic, cycloaliphatic or aromatic resins (including C5 aliphatic resins and C5/C9 aliphatic/aromatic resins); hydrogenated hydrocarbon resins; terpene-phenol resins and mixtures thereof.

Tackifiers may be effectively employed in combination with the patches used in accordance with the present invention and particularly in combination with the hydrogel layer or any PSA so employed.

### Permeable Inner Membrane

Having a permeable inner membrane on the inner surface of the hydrogel layer is a preferred feature of the first aspect of the present invention (*vide supra*)*.* Such an inner membrane is not to be considered a feature of the hydrogel layer *per se,* but as an additional feature.

It is preferred that the permeable inner membrane is breathable, particularly in combination with the hydrogel layer and backing layer being breathable.

It is preferred that the permeable inner membrane is flexible, for comfort in use reasons.

The permeable inner membrane is preferably a knitted, woven or non-woven structure forming a gauze, mesh or tulle.

In preferred embodiments, the permeable inner membrane is coated with a plastic porous film such as Telfa, which offers the additional benefit of allowing the patch to be more easily detached from the skin surface.

The permeable inner membrane does not occlude the skin. It is a thin film, which acts as a pliable barrier between the skin and the hydrogel layer and enhances comfort in wear.

Suitable materials for the permeable inner membrane include textiles such as polyester, rayon, cottons or mixtures such as polycotton. Other suitable materials are synthetic, porous polymer films, such as polyurethane, cellulose acetate, nitrocellulose, cellulose esters, polysulfone, polyether sulfone, polyacrylonitrile, polyamide, polyimide, polyethylene, polypropylene, polytetrafluoroethylene, polyvinylidene fluoride or polyvinylchloride.

### Benefit Agent

In certain preferred embodiments, a benefit agent is embedded in the hydrogel layer, said benefit agent being released when the patch is applied to the skin. In such embodiments, the release of the benefit agent may be triggered by the adsorption of sweat and/or delayed by its presence in the hydrogel layer..

The benefit agent may also be released by other physiological triggers such as body temperature or movement.

In particularly preferred embodiments, the benefit agent is a fragrance. In such embodiments, the fragrance can be made longer lasting by virtue of its release being delayed. In addition, by having the release triggered by means of perspiration absorption (for example) the body needs can be fragranced when it needs it most (i.e. when it is perspiring).

The fragrance employed may be any of those known in the art, including encapsulated fragrances which can give the benefit of delayed release beyond any such effect caused by the hydrogel layer.

The benefit agent may additionally or alternatively be an agent delivering a benefit relating to skin appearance or skin health. The benefit agent may be selected from skin tone promoters, skin barrier promoters, skin lightening agents, moisturisers (including humectants and emollients), skin cooling agents, anti-inflamatories or anti-bacterial agents, as well as or instead of fragrances. Benefit agents that improve skin health are particularly preferred.

The benefit agent may be embedded in the hydrogel layer by any suitable means, including both before and/or after polymerisation. In some embodiments, the benefit agent will be embedded before polymerisation is complete, in particular before the base polymer is crosslinked. This can offer the benefit of evenly embedding the benefit agent in the hydrogel layer. When this method is employed using fragrance as the benefit agent, photo-inititation and/or UV curing of the hydrogel is preferred, as this form of initiation can minimise damge to the fragrance.

In other embodiments, the benefit agent will be embedded in the hydrogel layer after polymerisation and cross-linking is complete. This is a preferred method when the benefit agent would otherwise be damaged by the polymerisation and/or crosslinking reaction.

The benefit agent is preferably added to the hydrogel layer as a solution in a carrier fluid. The carrier fluid is often present in a lesser quantity than the benefit agent when the benefit agent is a fragrance and may be termed a fragrance diluent in such circumstance. When employed, the fragrance is present in the hydrogel at a preferred level of from 0.1 to 10% by weight and a more preferred level of from 0.5 to 5% by weight of the hydrogel.

### Examples

The following examples are provided to better illustrate the invention and are not to be considered limiting.

Figure 1 shows a cross-section through a patch (1) suitable for use in accordance with the invention.

Figure 2 shows how a patch may be located in the underarm region (axilla) and shows a top view of the patch (1).

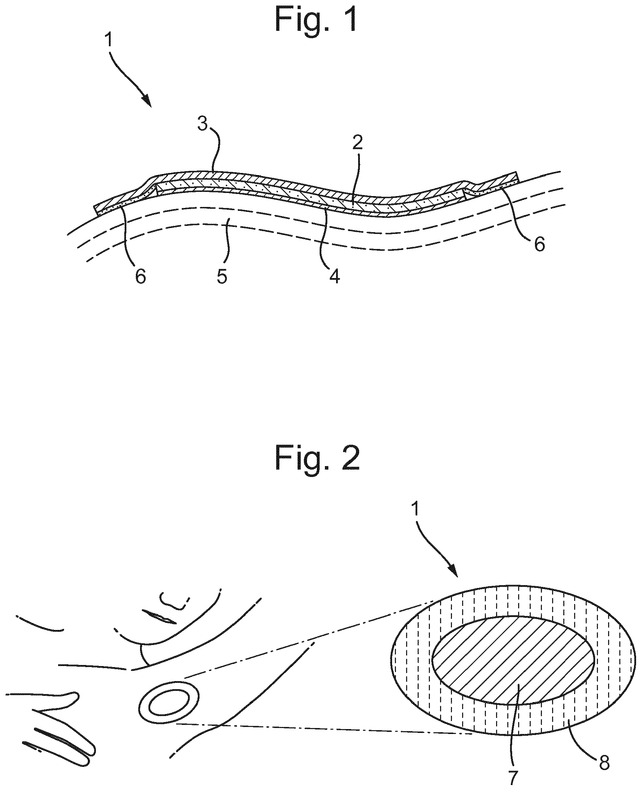

Figure 1 shows that the patch (1) comprises a hydrogel layer (2) sandwiched between a backing layer (3) and a permeable inner membrane (4) that contacts the skin (5). In this embodiment, each of the components is sufficiently flexible to allow the patch (1) as a whole to flex with the movement of the skin of the body to which it is attached.

Figure 1 further illustrates the backing layer (3) overlapping the hydrogel layer (2) and making contact with the skin (5) around the periphery of the hydrogel layer (2). The backing layer (3) adheres to the skin (5) in this peripheral region by means of a pressure sensitive adhesive (6) applied on the inner surface of the backing layer (3) around its periphery.

In Figure 2, the top view of the patch (1) illustrates a central region (7) and a peripheral region (8) of the patch. In the central region (7), the permeable inner membrane (4), hydrogel layer (2) and backing layer (3) lie one or top of the other. In the peripheral region (8), the backing layer (3) is stuck to the skin (5) by the pressure sensitive adhesive (6).

### Experiments

PolyAMPS hydrogel samples were synthesised using the following method. A batch of reaction mixture was prepared from 2-acrylamido-2-methylpropane sulfonic acid sodium salt (NaAMPS, 50% by weight in water, 46.4g in total), poly(ethylene glycol) diacrylate (PEGDA, Mₙ ~ 575 g mol⁻¹, 0.1077g), water (30.53g) and the photo-initiator Irgacure 1173 (2-hydroxy-2-methylpropiophenone, 0.1 ml of a 10% aqueous solution). 3 ml samples were transferred to individual moulds and then photo-cured using high intensity UV radiation from a Light Hammer^{®} from Fusion UV Systems Corp.

Sweat patches according to the invention (comprising a polyAMPS hydrogel layer as prepared above) were applied to the underarms of human volunteers. Following vigorous sporting activity, the patches were collected and extracted. Analysis of the extracts was by GC using a Shimadzu GC2014 GC. The retention time of the peaks in the worn sample were identified and compared with known retention times of volatile compounds tested. Linear regression analysis was used to determine the concentration based on pre-prepared calibration curves. GC analysis of the extracts revealed significant levels of acetic acid and propionic acid - both known malodourous components of human perspiration as typically exuded from the underarm regions. This is evidence of the absorption and removal of volatile malodourous materials by the present invention and hence evidence for the deodorancy benefit that it can deliver.

## Claims

1. A method of capturing perspiration from the human skin, said method comprising the topical application of a patch comprising an absorbent hydrogel layer and a backing sheet adhering to it, the perspiration being absorbed into the hydrogel layer and thereby trapped, wherein the hydrogel layer comprises a hydrophilic polymer which is an addition polymer having pendant hydrophilic groups.

2. A method according claim 1, wherein the hydrophilic polymer is poly(2-hydroxyethylmethacrylate) or a metal salt of poly(2-acrylo-2-methyl-1-propanesulfonic acid).

3. A method according to claim 2, wherein the hydrophilic polymer is a metal salt of poly(2-acrylo-2-methyl-1-propanesulfonic acid) crosslinked with poly(ethylene glycol) diacrylate.

4. A method according to any of preceding claims, wherein the backing sheet comprises a pressure sensitive adhesive.

5. A method according to any of preceding claims, wherein the absorbent hydrogel layer comprises a fragrance and the method also involves the release of said fragrance.

6. A method according to any of the preceding claims, wherein the absorbent hydrogel layer comprises a skin health promoter and the method also involves the release of said skin health promoter.

7. A method according to any of preceding claims which also captures volatile malodourous materials from the human skin.

## Patentansprüche

1. Verfahren zum Auffangen von Schweiß der menschlichen Haut, wobei das Verfahren die topische Anwendung eines Pflasters umfasst, das eine absorbierende Hydrogelschicht und eine daran haftende Trägerschicht umfasst, wobei der Schweiß von der Hydrogelschicht absorbiert und dadurch eingefangen wird, wobei die Hydrogelschicht ein hydrophiles Polymer umfasst, das ein Additionspolymer mit hydrophilen Seitengruppen ist.

2. Verfahren nach Anspruch 1, wobei das hydrophile Polymer Poly(2-hydroxyethylmethacrylat) oder ein Metallsalz von Poly(2-acrylo-2-methyl-1-propansulfonsäure) ist.

3. Verfahren nach Anspruch 2, wobei das hydrophile Polymer ein Metallsalz der Poly(2-acrylo-2-methyl-1-propansulfonsäure) ist, das mit Poly(ethylenglycol) diacrylat vernetzt ist.

4. Verfahren nach einem der vorhergehenden Ansprüche, wobei die Trägerschicht einen druckempfindlichen Klebstoff umfasst.

5. Verfahren nach einem der vorhergehenden Ansprüche, wobei die absorbierende Hydrogelschicht einen Duftstoff umfasst und das Verfahren außerdem die Freisetzung des Duftstoffs einbezieht.

6. Verfahren nach einem der vorhergehenden Ansprüche, wobei die absorbierende Hydrogelschicht einen Hautgesundheitsförderer umfasst und das Verfahren außerdem die Freisetzung des Hautgesundheitsförderers einbezieht.

7. Verfahren nach einem der vorhergehenden Ansprüche, das außerdem übelriechende flüchtige Materialien der menschlichen Haut auffängt.

## Revendications

1. Procédé pour capturer la transpiration à partir de la peau humaine, ledit procédé comprenant l'application topique d'un timbre comprenant une couche d'hydrogel absorbant et une feuille d'envers y adhérant, la transpiration étant absorbée dans la couche d'hydrogel et ainsi piégée, dans lequel la couche d'hydrogel comprend un polymère hydrophile qui est un polymère d'addition ayant des groupes hydrophiles pendants.

2. Procédé selon la revendication 1, dans lequel le polymère hydrophile est le poly(méthacrylate de 2-hydroxyéthyle) ou un sel métallique de poly(acide 2-acrylo-2-méthyl-1-propanesulfonique).

3. Procédé selon la revendication 2, dans lequel le polymère hydrophile est un sel métallique de poly(acide 2-acrylo-2-méthyl-1-propanesulfonique) réticulé avec du diacrylate de polyéthylèneglycol.

4. Procédé selon l'une quelconque des revendications précédentes, dans lequel la feuille d'envers comprend un adhésif sensible à la pression.

5. Procédé selon l'une quelconque des revendications précédentes, dans lequel la couche d'hydrogel absorbant comprend un parfum, lequel procédé implique également la libération dudit parfum.

6. Procédé selon l'une quelconque des revendications précédentes, dans lequel la couche d'hydrogel absorbant comprend un promoteur de santé de la peau, lequel procédé implique également la libération dudit promoteur de santé de la peau.

7. Procédé selon l'une quelconque des revendications précédentes, qui capture aussi les matières malodorantes volatiles à partir de la peau humaine.
